# EUROPEAN PATENT APPLICATION

(11) **EP 1 627 644 A1**
(43) Date of publication of application: **22.02.2006**
(21) Application number: 04734601.0
(22) Date of filing: 24.05.2004
(51) Int. Cl.: A61K 47/10, A61K 45/00, A61K 47/14, A61K 31/05, A61K 31/192, A61K 31/196, A61K 31/235, A61K 31/381, A61K 31/40, A61K 31/403, A61K 31/416, A61K 31/423, A61K 31/53, A61K 31/5415, A61P 17/00, A61P 29/00

(54) **PREPARTAION FOR EXTERNAL PERCUTANEOUS ADMINISTRATION CONTAINING NON-STEROIDAL ANTI-INFLAMMATORY DRUG AND INTERLEUKIN-1a PRODUC TION INHIBITOR**

(30) Priority: 23.05.2003 JP 2003146646
(71) Applicant: HISAMITSU PHARMACEUTICAL CO. INC., Tosu-shi, Saga-ken 841-0017 (JP)
(72) Inventor: IKESUE, A., Tsukuba Hisamitsu Pharma. Co., Inc, Tsukuba-shi, Ibaraki 3050856 (JP); YOSHITAKE, K., Tsukuba Hisamitsu Pharma. Co. Inc, Tsukuba-shi, Ibaraki 3050856 (JP); ATARASHI, K., Tsukuba Hisamitsu Pharma Co. Inc., Tsukuba-shi, Ibaraki 3050856 (JP); IKESUE, K., Tsukuba HISAMITSUPharma Co. Inc, Tsukuba-shi, Ibaraki 3050856 (JP); SAKAI, M., Tsukuba Hisamitsu Pharma co. Inc, Tsukuba-shi, Ibaraki 3050856 (JP); REDDY, A., Ca. Lab. Hisamitsu Pharma co. inc, Carlsbad, CA 92008 (US); MOTOKI, Y., CA. Lab. Hisamitsu Pharma co. inc, Carlsbad, CA 92008 (US); VEERAPANENI, Dange, Carlsbad, CA 92008 (US)
(74) Representative: Grünecker, Kinkeldey, Stockmair & Schwanhäusser Anwaltssozietät
(86) International application number: PCT/JP2004/007403
(87) International publication number: WO 2004/112837

(57) **Abstract**

A transdermal formulation for external application comprising a non-steroidal anti-inflammatory analgesic, an alkyl ester of gallic acid, and a phenolic radical scavenger having a branched-chain lower alkyl group.

An interleukin-1α production inhibitor consisting of a phenolic radical scavenger having a branched-chain lower alkyl group, and/or an alkyl ester of gallic acid.

## Description

### Technical Field

The present invention relates to a transdermal formulation for external application containing a non-steroidal anti-inflammatory analgesic, and to an interleukin-1α (IL-1α) production inhibitor.

### Background Art

Non-steroidal anti-inflammatory analgesics such as ketoprofen have excellent anti-inflammatory and analgesic actions, and are therefore added as drug components to various forms of transdermal formulations for external application, such as patches (poultices, plasters and the like), gels, creams, ointments and liniments.

On the other hand, non-steroidal anti-inflammatory analgesics such as ketoprofen are also known to be relatively unstable with respect to light. Japanese Patent Application Laid-Open SHO 60-155111 discloses that ketoprofen is stabilized by addition of an ultraviolet absorbent such as a p-aminobenzoic acid derivative, a cinnamic acid derivative, a benzophenone derivative, a coumarin derivative or an amino acid compound to a ketoprofen-containing transdermal formulation for external application. It is further disclosed that addition of an antioxidant such as ascorbyl stearate, sodium ascorbate, tocopherol, nordihydroguaiaretic acid, dibutylhydroxytoluene, butylhydroxyanisole, *tert*-butylhydroquinone gallic acid ester or 1-oxo-3-methyl-4-isopropylbenzene in combination with the aforementioned ultraviolet absorbent is also effective for stabilizing ketoprofen.

### Disclosure of the Invention

However, even the transdermal formulations for external application described in Japanese Patent Application Laid-Open SHO 60-155111 can potentially, though rarely, cause photosensitivity. It has therefore been desirable to develop transdermal formulations for external application that can more reliably prevent photosensitivity while exhibiting anti-inflammatory analgesic effects.

The present invention has been accomplished in light of the problems of the prior art described above, and its object is to reliably prevent photosensitivity in a transdermal formulation for external application comprising a non-steroidal anti-inflammatory analgesic as an active ingredient which can potentially induce photosensitivity.

As a result of much avid research directed toward achieving the aforestated object, the present inventors have discovered that, in a transdermal formulation for external application comprising a non-steroidal anti-inflammatory analgesic as an active ingredient which can potentially induce photosensitivity, addition of both an alkyl ester of gallic acid and a phenolic radical scavenger having a branched-chain lower alkyl group, in combination, notably inhibits photosensitivity through their synergistic action. The present inventors also discovered that such a phenolic radical scavenger having a branched-chain lower alkyl group, and an alkyl ester of gallic acid together have an inhibitory action on IL-1α production.

Specifically, the present invention provides, firstly, a transdermal formulation for external application comprising a non-steroidal anti-inflammatory analgesic, an alkyl ester of gallic acid, and a phenolic radical scavenger having a branched-chain lower alkyl group.

Drug-induced photosensitivity is generally classified as either allergic or phototoxic (resulting from free radicals or peroxides produced during photodecomposition of a drug). As a result of studying the causes of photosensitivity induced by ketoprofen-containing formulations, the present inventors have found that free radicals generated by decomposition of a non-steroidal anti-inflammatory analgesic due to light exposure are strongly implicated as a cause of photosensitivity, and that photosensitivity resulting from free radicals generated by a small degree of photodecomposition of a non-steroidal anti-inflammatory analgesic is not sufficiently inhibited in conventional transdermal formulations for external application such as described in Japanese Patent Application Laid-Open SHO 60-155111. The present inventors also found, after much diligent research aimed at achieving sufficient inhibition of photosensitivity caused by such free radicals, that if both an alkyl ester of gallic acid and a phenolic radical scavenger having a branched-chain lower alkyl group are added in combination to a transdermal formulation for external application comprising a non-steroidal anti-inflammatory analgesic, the phototoxicity-reducing action is synergistically reinforced and photosensitivity is notably inhibited. No such synergistic effect is observed if a nonylic vanillylamide with no branched-chain lower alkyl group is used, even though it is a phenolic radical scavenger. The present inventors therefore conjectured that the phototoxicity-reducing action is reinforced by the intermolecular CH/π interaction between the π electrons of the phenyl group of the alkyl ester of gallic acid and the branched-chain lower alkyl group of the phenolic radical scavenger. Also, since no such synergistic effect is seen when adding a combination of two phenolic radical scavengers with branched-chain lower alkyl groups (BHA and BHT), it is believed that steric hindrance caused by the branched-chain lower alkyl groups affects the intermolecular CH/π interaction.

Thus, the phototoxicity-reducing action of the transdermal formulation for external application of the invention is a synergistic action exerted specifically when an alkyl ester of gallic acid is combined with a phenolic radical scavenger having a branched-chain lower alkyl group, and the action is exerted more prominently in the skin. Thus, a transdermal formulation for external application comprising these components and a non-steroidal anti-inflammatory analgesic can exhibit an anti-inflammatory analgesic effect while more reliably preventing photosensitivity, allowing its use as a highly safe pharmaceutical.

The present invention also provides an IL-1α production inhibitor consisting of a phenolic radical scavenger having a branched-chain lower alkyl group, and/or an alkyl ester of gallic acid. Such an IL-1α production inhibitor may likewise be used as a component in a transdermal formulation for external application containing a non-steroidal anti-inflammatory analgesic. Thus, the invention further provides a transdermal formulation for external application comprising such an IL-1α production inhibitor and a non-steroidal anti-inflammatory analgesic.

IL-1α is a cytokine produced in many different types of cells (macrophages, vascular endothelial cells, keratinocytes, etc.), and it is known to have an inflammation-provoking action. Thus, an IL-1α production inhibitor according to the invention should exhibit an effect of inhibiting inflammatory responses in the body. When used as a component in a transdermal formulation for external application comprising a non-steroidal anti-inflammatory analgesic, it should inhibit photosensitivity (dermatitis) induced by the non-steroidal anti-inflammatory analgesic, via an inhibitory action on IL-1α production in keratinocytes in the skin, and the aforementioned phototoxicity-reducing action.

### Brief Description of the Drawings

Fig. 1 is a graph showing the relationship between propyl gallate concentration and IL-1α concentration.
Fig. 2 is a graph showing the relationship between BHT concentration and IL-1α concentration.

### Best Modes for Carrying Out the Invention

Preferred embodiments of the transdermal formulation for external application and IL-1α production inhibitor of the invention will now be explained in detail. Throughout the present specification, "branched-chain lower alkyl group" refers to a C3-6 alkyl group with a branched chain, and "lower alcohol" refers to a C1-5 alcohol.

First, embodiments of the transdermal formulation for external application comprising a non-steroidal anti-inflammatory analgesic, an alkyl ester of gallic acid, and a phenolic radical scavenger having a branched-chain lower alkyl group will be explained.

As alkyl esters of gallic acid to be used in the transdermal formulation for external application of the invention there may be mentioned esters of gallic acid and lower alcohols (methyl gallate, ethyl gallate, propyl gallate, butyl gallate, etc.), among which propyl gallate is preferred. Such alkyl esters of gallic acid may be used alone or in combinations of two or more.

There are no particular restrictions on the content of the alkyl ester of gallic acid in the transdermal formulation for external application of the invention, but it is preferably 0.001-0.2% by weight based on the total formulation. If the content of the alkyl ester of gallic acid is less than the lower limit the phototoxicity-reducing action tends not to be adequately exerted, while if it is greater than the upper limit, a greater potential will exist for sensitization by the alkyl ester of gallic acid.

As radical scavengers to be used in the transdermal formulation for external application of the invention there may be mentioned radical scavengers which are phenol derivatives with branched-chain lower alkyl groups (*tert*-butyl, isopropyl, *tert*-pentyl, neopentyl, isohexyl, etc.), among which *tert*-butylhydroxyanisole (2-*tert*-butyl-4-oxyanisole, BHA), di-*tert*-butylhydroxytoluene (2,6-di-*tert*-butyl-p-cresol, BHT) and thymol (6-isopropyl-m-cresol) are preferred. Such radical scavengers may be used alone or in combinations of two or more.

There are no particular restrictions on the content of the phenolic radical scavenger in the transdermal formulation for external application of the invention, but it is preferably 0.01-10% by weight based on the total formulation. If the content of the phenolic radical scavenger is less than the lower limit the phototoxicity-reducing action tends not to be adequately exerted, while if it is greater than the upper limit, a greater potential will exist for dermatitis or hypersensitivity caused by the phenolic radical scavenger.

As explained above, it is an object of the invention to reliably prevent photosensitivity in a transdermal formulation for external application comprising a non-steroidal anti-inflammatory analgesic as an active ingredient. There are no particular restrictions on the non-steroidal anti-inflammatory analgesic so long as it can potentially cause photosensitivity, and as examples there may be mentioned ketoprofen, tiaprofen, suprofen, tolmetin, loxoprofen, zaltoprofen, carprofen, benoxaprofen, pranoprofen, fluribiprofen, felbinac, indomethacin, mefenamic acid, nabumetone, piroxicam, ampiroxicam, lomoxicam, meloxicam, mofezolac, benzydamine, naproxen, diclofenac, ibuprofen, diflunisal, azapropazone, tiaramide hydrochloride, salicylic acid, methyl salicylate, etodolac, celecoxib, rofecoxib, valdecoxib and parecoxib. Ketoprofen, tiaprofen, suprofen and tolmetin, which have a benzophenone skeleton or a benzophenone-like skeleton, are preferred among these as non-steroidal anti-inflammatory analgesics in the transdermal formulation for external application of the invention, because of their relatively high potential to cause photosensitivity, and ketoprofen which has a benzophenone skeleton is even more preferred. Such non-steroidal anti-inflammatory analgesics may be used alone or in combinations of two or more.

There are no particular restrictions on the content of the non-steroidal anti-inflammatory analgesic in the transdermal formulation for external application of the invention, but it is preferably 0.1-10% by weight based on the total formulation. If the content of the non-steroidal anti-inflammatory analgesic is less than the lower limit the anti-inflammatory analgesic action tends not to be adequately exerted, while if it is greater than the upper limit, a greater potential will exist for side-effects of the non-steroidal anti-inflammatory analgesic (not being limited to photosensitivity).

The transdermal formulation for external application of the invention preferably contains, in addition to the aforementioned essential components (the non-steroidal anti-inflammatory analgesic, alkyl ester of gallic acid, and phenolic radical scavenger), also a base selected depending on the dosage form of the formulation. As dosage forms of the transdermal formulation for external application of the invention there may be mentioned patches (poultices, plasters and the like), gels, creams, ointments and liniments. Preferred embodiments of the transdermal formulation for external application of the invention for each of these dosage forms will now be explained in detail.

A poultice will be explained first. There are no particular restrictions on poultice bases used for a poultice according to the invention, and any commonly used one may be selected. As examples of components to be contained in such poultice bases there may be mentioned thickening agents (synthetic water-soluble polymers (sodium polyacrylate, polyacrylic acid, polyvinyl alcohol, polyvinylpyrrolidone, polyethylene oxide, polyvinyl methacrylate, etc.), natural substances (gum arabic, starch, gelatin, etc.), methyl cellulose, hydroxypropyl cellulose, alginic acid, sodium alginate, ammonium alginate, carboxymethylcellulose sodium, etc.), humectants (urea, glycerin, propylene glycol, butylene glycol, sorbitol, etc.), fillers (kaolin, zinc oxide, talc, titanium, bentonite, epoxy resins, organic acids (citric acid, tartaric acid, maleic acid, maleic anhydride, succinic acid, etc.), calcium, magnesium, aluminum, etc.), water, solubilizers (propylene carbonate, crotamiton, diisopropyl adipate, etc.), tackifiers (rosins, ester gums, polybutene, polyacrylic esters, etc.), rash-preventing agents (diphenhydramine hydrochloride, chlorpheniramine maleate, glycyrrhizinic acid, dexamethasone, betamethasone, fluocinolone acetonide, etc.) and other additives (salicylic acid, methyl salicylate, glycol salicylate, 1-menthol, camphor, nonylic vanillylamide, capsicum extract, peppermint oil, Azone^{R}, etc.). A poultice according to the invention can be obtained by mixing the aforementioned essential components with a poultice base composed of a mixture of various components selected from the above.

A suitable production example (formulation example) for a poultice will now be described. First, 0.1-10 parts by weight of the non-steroidal anti-inflammatory analgesic, 0.001-0.2 part by weight of the alkyl ester of gallic acid and 0.01-10 parts by weight of the phenolic radical scavenger are mixed with 0.5-8 parts by weight of a solubilizer to obtain mixture A. Separately, 5-20 parts by weight (preferably 10-15 parts by weight) of a thickening agent is mixed with 5-40 parts by weight of a humectant and 10-80 parts by weight of water, and then up to 20 parts by weight of a filler is added to obtain kneaded mixture B. Mixture A is then mixed with kneaded mixture B and the obtained kneaded mixture is spread onto a support, after which a release cover is attached thereon to obtain a poultice of the invention. The support used may be an elastic or non-elastic support. As an example of the support there may be mentioned a support made of cloth, nonwoven fabric, polyurethane, polyester, polyvinyl acetate, polyvinylidene chloride, polyethylene, polyethylene terephthalate, aluminum sheet, or a composite thereof. As an example of the release cover there may be mentioned a release cover made of polyethylene, polypropylene, polyvinyl chloride, polyester, polyvinylidene chloride or silicone-treated paper.

A plaster will now be described. There are no particular restrictions on plaster bases used for a plaster according to the invention, and any commonly used one may be selected. As examples of components to be contained in such plaster bases there may be mentioned polymer bases (acrylic-based compositions which are copolymers with vinyl monomers (methacrylic esters, acrylonitrile, vinyl acetate, vinyl propionate, etc.), silicone resins, polyisoprene rubber, polyisobutylene rubber, natural rubber, acrylic rubber, styrenebutadiene-styrene block copolymer, styrene-isoprene-styrene block copolymer, etc.), fats/oils or higher fatty acids (almond oil, olive oil, camellia oil, apricot oil, peanut oil, olein oil, liquid paraffin, polybutene, etc.), tackifiers (rosins, rosin-modified maleic acid, hydrogenated rosin esters, etc.), fatty acid metal salts (zinc undecylenate, zinc stearate, calcium stearate, aluminum stearate, magnesium stearate, sodium stearate, zinc palmitate, zinc myristate, magnesium myristate, sodium laurate, zinc laurate), rash-preventing agents, and other additives (salicylic acid, methyl salicylate, glycol salicylate, dl-camphor, 1-menthol, nonylic vanillylamide, capsicum tincture, peppermint oil, crotamiton, Azone^{R}, etc.). A plaster according to the invention can be obtained by mixing the aforementioned essential components with a plaster base comprising a mixture of various components selected from the above.

A suitable production example (formulation example) for a plaster will now be described. For production by a hot melt method, first a mixing apparatus such as a kneader or mixer is used for mixing of 5-40 parts by weight of a polymer base, 20-70 parts by weight of a fat/oil or higher fatty acid, 10-40 parts by weight of a tackifier and 0.1-10 parts by weight of a fatty acid metal salt at 120-160°C. Next, 0.1-10 parts by weight of the non-steroidal anti-inflammatory analgesic, 0.001-0.2 part by weight of the alkyl ester of gallic acid and 0.01-10 parts by weight of the phenolic radical scavenger are mixed. After spreading the obtained mixture onto a support, a release cover is attached thereon to obtain a plaster according to the invention, or alternatively, after spreading the obtained mixture onto a release-treated paper or film, it is used to cover a support and transferred thereto by pressure bonding to obtain a plaster according to the invention. For production by a solvent method, first a mixing apparatus such as an explosion-proof mixer is used for dissolution of the components into a solvent such as toluene, hexane or methylene chloride. The obtained solution is then spread onto a release-treated paper or film, and the solvent is distilled off with a drier, after which this is used to cover a support and transferred thereto by pressure bonding to obtain a plaster according to the invention. A plaster according to the invention may also be obtained by spreading the solution onto a support and distilling off the solvent, and then attaching a release cover thereon. The support used may be an elastic or non-elastic support. As an example of the support there may be mentioned a support made of cloth, nonwoven fabric, polyurethane, polyester, polyvinyl acetate, polyvinylidene chloride, polyethylene, polyethylene terephthalate, aluminum sheet, or a composite thereof. As an example of the release cover there may be mentioned a release cover made of polyethylene, polypropylene, polyvinyl chloride, polyester, polyvinylidene chloride or silicone-treated paper.

An ointment will now be described. There are no particular restrictions on ointment bases used for an ointment according to the invention, and any commonly used one may be selected. As examples of components to be contained in such ointment bases there may be mentioned higher fatty acids or their esters (adipic acid, myristic acid, palmitic acid, stearic acid, oleic acid, adipic esters, myristic esters, palmitic esters, diethyl sebacate, hexyl laurate, cetyl isooctanate, etc.), waxes (spermaceti, beeswax, ceresin, etc.), surfactants (polyoxyethylene alkyl ether phosphates, etc.), higher alcohols (cetanol, stearyl alcohol, cetostearyl alcohol, etc.), silicone oils (dimethylpolysiloxane, methylphenylpolysiloxane, glycol methylpolysiloxane, silicone glycol polymers, etc.), hydrocarbons (hydrophilic vaseline, white vaseline, purified lanolin, liquid paraffin, etc.), water, absorption enhancers (propylene carbonate, diisopropyl adipate, crotamiton, Azone^{R}, etc.), humectants (glycerin, propylene glycol, butylene glycol, sorbitol; etc.), rash-preventing agents, and other additives (salicylic acid, methyl salicylate, glycol salicylate, 1-menthol, camphor, peppermint oil, etc.). An ointment according to the invention can be obtained by mixing the aforementioned essential components with an ointment base comprising a mixture of various components selected from the above.

A suitable production example (formulation example) for an ointment will now be described. First, 0.1-10 parts by weight of the non-steroidal anti-inflammatory analgesic, 0.001-0.2 part by weight of the alkyl ester of gallic acid and 0.01-10 parts by weight of the phenolic radical scavenger are mixed with 5-15 parts by weight of a higher fatty acid ester and 1-10 parts by weight of a surfactant, either at room temperature or under heating. Next, 4-10 parts by weight of a wax and 50-90 parts by weight of a hydrocarbon are added thereto, and the mixture is heated and kept at 50-100°C. When all the components have dissolved to produce a transparent solution, it is blended with a homomixer, and the obtained mixture is stirred while lowering the temperature to room temperature, to obtain an ointment according to the invention.

A gel will now be described. There are no particular restrictions on gel bases used for a gel according to the invention, and any commonly used one may be selected. As examples of components to be contained in such gel bases there may be mentioned lower alcohols (ethanol, isopropyl alcohol, etc.), water, gelling agents (carboxyvinyl polymer, hydroxyethyl cellulose, hydroxypropyl cellulose, methyl cellulose, ethyl cellulose, carboxymethyl cellulose, propyleneglycol alginate, etc.), neutralizing agents (triethanolamine, diisopropylalcohol amines, sodium hydroxide, etc.), surfactants (sorbitan sesquioleate, sorbitan trioleate, sorbitan monooleate, sorbitan monostearate, sorbitan monolaurate, polyethylene glycol monostearate, polyoxyethylene nonyl phenyl ether, polyoxyethylene cetyl ether, polyoxyethylene lauryl ether, etc.), absorption enhancers (propylene carbonate, diethyl sebacate, diisopropyl adipate, crotamiton, Azone^{R}, propylene glycol, etc.), rash-preventing agents, and other additives (salicylic acid, methyl salicylate, glycol salicylate, 1-menthol, camphor, peppermint oil, etc.). A gel according to the invention can be obtained by mixing the aforementioned essential components with a gel base comprising a mixture of various components selected from the above.

A suitable production example (formulation example) for a gel will now be described. First, 0.5-5 parts by weight of a gelling agent is added to 55 parts by weight of water for swelling, to obtain swelled mixture A. Separately, 0.1-10 parts by weight of the non-steroidal anti-inflammatory analgesic, 0.001-0.2 part by weight of the alkyl ester of gallic acid and 0.01-10 parts by weight of the phenolic radical scavenger are mixed with 0.1-10 parts by weight of a solubilizer, and this mixture is dissolved in a mixture of up to 40 parts by weight of a glycol and up to 60 parts by weight of a lower alcohol to obtain solution B. Solution B is then added to swelled mixture A, and then the neutralizing agent is added to adjust the pH to 4-7 to obtain a gel according to the invention.

A cream will now be described. There are no particular restrictions on cream bases used for a cream according to the invention, and any commonly used one may be selected. As examples of components to be contained in such cream bases there may be mentioned higher fatty acid esters (myristic esters, palmitic esters, diethyl sebacate, hexyl laurate, cetyl isooctanate, etc.), lower alcohols (ethanol, isopropyl alcohol, etc.), carbohydratres (liquid paraffin, squalane, etc.), polyhydric alcohols (propylene glycol, 1,3-butylene glycol, etc.), higher alcohols (2-hexyldecanol, cetanol, 2-octyldodecanol, etc.), emulsifiers (polyoxyethylene alkyl ethers, fatty acid esters, polyethylene glycol fatty acid esters, etc.), preservatives (paraoxybenzoic acid esters, etc.), absorption enhancers (propylene carbonate, diethyl sebacate, diisopropyl adipate, crotamiton, Azone^{R}, etc.), rash-preventing agents, and other additives (salicylic acid, methyl salicylate, glycol salicylate, 1-menthol, camphor, peppermint oil, etc.). A cream according to the invention can be obtained by mixing the aforementioned essential components with a cream base comprising a mixture of various components selected from the above. Also, a gel cream (a dosage form having properties intermediate between a cream and a gel) according to the invention can be obtained by adding a gelling agent (carboxyvinyl polymer, hydroxyethyl cellulose, hydroxypropyl cellulose, ethyl cellulose, carboxymethyl cellulose, etc.) to the aforementioned cream, and further adjusting the pH to 4-8 (preferably 5-6.5) by addition of a neutralizing agent (diisopropylalcohol amines, triethanolamine, sodium hydroxide, etc.).

A suitable production example (formulation example) for a gel cream will now be described. First, 0.1-10 parts by weight of the non-steroidal anti-inflammatory analgesic, 0.001-0.2 part by weight of the alkyl ester of gallic acid and 0.01-10 parts by weight of the phenolic radical scavenger are dissolved in a mixture of up to 25 parts by weight of a higher fatty acid ester and up to 40 parts by weight of a lower alcohol, and then up to 0.5 part by weight of a preservative and up to 5 parts by weight of an emulsifier are added to obtain mixture A. Separately, a gelling agent is added to water at a concentration of 0.5-5% by weight for swelling to obtain swelled mixture B. Swelled mixture B is then added to mixture A, and after uniform emulsification with a homomixer, a neutralizing agent is added to the obtained emulsion to adjust the pH to 4-8, in order to obtain a gel cream according to the invention.

A liniment will now be described. There are no particular restrictions on liniment bases used for a liniment according to the invention, and any commonly used one may be selected. As examples of such liniment bases there may be mentioned mixtures composed of 10-70 parts by weight of an alcohol (a monohydric alcohol (ethanol, propanol, isopropyl alcohol, etc.), polyhydric alcohol (polyethylene glycol, propylene glycol, butylene glycol, etc.), or the like), up to 55 parts by weight of water, up to 60 parts by weight of a fatty acid ester (an ester of adipic acid, sebacic acid, myristic acid, etc.) and up to 10 parts by weight of a surfactant (polyoxyethylene alkyl ether). By adding 0.1-10 parts by weight of the non-steroidal anti-inflammatory analgesic, 0.001-0.2 part by weight of the alkyl ester of gallic acid and 0.01-10 parts by weight of the phenolic radical scavenger to such a liniment base, it is possible to obtain a liniment according to the invention. If necessary, for example, neutralizing agents (for pH adjustment), tackifiers (methyl cellulose, carboxyvinyl polymer, hydroxypropyl cellulose, etc.), rash-preventing agents, and other additives (salicylic acid, methyl salicylate, glycol salicylate, 1-menthol, camphor, peppermint oil, capsicum extract, nonylic vanillylamide, crotamiton, Azone^{R}, propylene carbonate, diisopropyl adipate, etc.) may also be added in the liniment of the invention.

The production example (formulation example) for a liniment as described above is merely an example, and a liniment of the invention may, of course, be obtained by any publicly known production method for liniments. For example, a liniment of the invention may be obtained by replacing the active ingredient of a publicly known liniment with the non-steroidal anti-inflammatory analgesic such as ketoprofen, and combining therewith the phenolic radical scavenger having a branched-chain lower alkyl group, and the alkyl ester of gallic acid.

Preferred embodiments of the transdermal formulation for external application of the invention for different dosage forms were explained above, but these dosage forms and production examples (formulation examples) are not intended to be limitative. Also, the order of mixing the components in the formulation are not particularly limited. For example, the active ingredient in a publicly known eye drop or aerosol may be replaced with 0.1-10 parts by weight of the non-steroidal anti-inflammatory analgesic, and then 0.001-0.2 part by weight of the alkyl ester of gallic acid and 0.01-10 parts by weight of the phenolic radical scavenger combined therewith, to obtain an eye drop or aerosol according to the invention.

The transdermal formulation for external application of the invention may also contain an antioxidant and/or an ultraviolet absorbent in addition to the components indicated in the aforementioned production examples (formulation examples). Preferred antioxidants include tocopherol and its ester derivatives, ascorbic acid, ascorbyl stearate, nordihydroguaiaretic acid and the like. Preferred ultraviolet absorbents include p-aminobenzoic acid derivatives, anthranilic acid derivatives, salicylic acid derivatives, coumarin derivatives, amino acid-based compounds, imidazoline derivatives, pyrimidine derivatives, dioxane derivatives and the like. The respective contents of such an antioxidant and such an ultraviolet absorbent are not particularly restricted, but are preferably 0-10% by weight and more preferably 0-5% by weight based on the total formulation.

Preferred embodiments of the IL-1α production inhibitor of the invention will now be explained.

As radical scavengers to be used in the IL-1α production inhibitor of the invention there may be mentioned radical scavengers which are phenol derivatives with branched-chain lower alkyl groups (*tert*-butyl, isopropyl, *tert*-pentyl, neopentyl, isohexyl, etc.), among which *tert*-butylhydroxyanisole (2-*tert*-butyl-4-oxyanisole, BHA), di-*tert*-butylhydroxytoluene (2,6-di-*tert*-butyl-p-cresol, BHT) and thymol (6-isopropyl-m-cresol) are preferred. Such radical scavengers may be used alone or in combinations of two or more.

As alkyl esters of gallic acid to be used in the IL-1α production inhibitor of the invention there may be mentioned esters of gallic acid and lower alcohols (methyl gallate, ethyl gallate, propyl gallate, butyl gallate, etc.), among which propyl gallate is preferred. Such alkyl esters of gallic acid may be used alone or in combinations of two or more.

The radical scavenger and the alkyl ester of gallic acid may be used either alone, or both in combination, in the IL-1α production inhibitor of the invention.

When the IL-1α production inhibitor of the invention is used as a component in a transdermal formulation for external application comprising a non-steroidal anti-inflammatory analgesic, its purpose is to inhibit photosensitivity due to the non-steroidal anti-inflammatory analgesic, and thus as embodiments thereof, embodiments in which the phototoxicity-reducing action is sufficiently exerted are preferred. That is, the embodiments of the transdermal formulation for external application comprising a non-steroidal anti-inflammatory analgesic, an alkyl ester of gallic acid, and a phenolic radical scavenger having a branched-chain lower alkyl group, as described above, are also preferred in this case.

### (Examples)

The present invention will now be explained in greater detail through examples and comparative examples, with the understanding that these examples are in no way limitative on the invention. Unless otherwise specified, the "%" values in the examples and comparative examples below are based on weight.

### (Examples 1-3): in vitro Phototoxicity test

The following experiment was conducted according to "Guidelines for basic biological tests of medical materials and devices", Part VII "Hemolysis Test". First, each of the test substances listed in Table 1 (Example 1), Table 2 (Example 2) and Table 3 (Example 3) {*tert*-butylhydroxyanisole (BHA), di-*tert*-butylhydroxytoluene (BHT), thymol, propyl gallate} and ketoprofen were dissolved in N,N-dimethylformamide (DMF) to 20-fold compared to the concentration shown in the tables (1000 µg/mL of ketoprofen). Also, after further diluting these solutions 5-fold in phosphate buffered saline (PBS), equal amounts of each test substance solution and the ketoprofen solution were combined. As a control, ketoprofen alone was dissolved in DMF to 1000 µg/mL, and diluted 10-fold with PBS to prepare a solution. These solutions were dispensed in 1 mL portions into a 24-well multiwell plate, and then 1 mL of PBS containing 40 µL of heparin-added rabbit venous blood (whole blood) per 5 mL was added to each to prepare reaction solutions.

An ultraviolet irradiating apparatus (UVP Lamp Fixture, UVP) equipped with two ultraviolet lamps (FL15BLB, Sankyo Denki) was used to irradiate the reaction solutions with 7.5 J/cm² UVA (ultraviolet rays of wavelength 320-400 nm) in an incubator set to 37°C, with the cover removed from the plate containing the reaction solutions (UVA is associated with drug-induced photosensitivity). During the ultraviolet irradiation, a 3 mm-thick glass plate was placed between the light source and the reaction solutions to cut ultraviolet rays of 320 nm wavelength and shorter. The incubation time was 60 minutes, including the irradiation time. After completion of the reaction, the solutions were centrifuged, the obtained 100 µL of supernatant (N=3) was transferred to a 96-well assay plate and the absorbance at 540 nm (Abs₅₄₀) was measured as an index of hemolysis. The effects of each of the test substances in Tables 1 to 3 on ketoprofen-associated photohemolysis were evaluated as the hemolysis inhibition (%) with respect to the control group, calculated using Abs₅₄₀. The obtained results are shown in Tables 1 to 3.

**(Table 1)**

| µg/mL | | BHA | | | | |
|---|---|---|---|---|---|---|
| | | 0 | 0.125 | 0.25 | 0.5 | 1 |
| Propyl gallate | 0 | 0.0 | 7.9 | 17.5 | 69.6 | 94.3 |
| | 0.5 | 25.5 | 51.1 | 74.2 | 90.7 | 95.0 |
| | 1 | 66.0 | 86.4 | 89.7 | 93.9 | 96.9 |
| | 2 | 88.9 | 90.5 | 93.1 | 95.1 | 98.6 |

**(Table 2)**

| µg/mL | | BHT | | | | |
|---|---|---|---|---|---|---|
| | | 0 | 0.125 | 0.25 | 0.5 | 1 |
| Propyl gallate | 0 | 0.0 | -3.7 | 9.6 | 49.6 | 88.9 |
| | 0.5 | 3.6 | 38.1 | 88.6 | 91.6 | 96.1 |
| | 1 | 45.4 | 86.9 | 91.2 | 95.9 | 98.5 |
| | 2 | 91.2 | 92.6 | 96.3 | 96.8 | 98.9 |

**(Table 3)**

| µg/mL | | Thymol | | | |
|---|---|---|---|---|---|
| | | 0 | 0.5 | 1 | 2 |
| Propyl gallate | 0 | 0.0 | 10.8 | 41.1 | 70.7 |
| | 0.5 | 2.1 | 61.4 | 76.0 | 91.0 |
| | 1 | 63.8 | 91.4 | 91.5 | 95.8 |
| | 2 | 88.0 | 95.8 | 94.7 | 97.5 |

As is clear from the results shown in Tables 1-3, combining an alkyl ester of gallic acid and BHA, BHT or thymol in a transdermal formulation for external application comprising ketoprofen as an active ingredient was confirmed to notably reduce phototoxicity through their synergistic action.

### (Examples 4-5 and Comparative Examples 1-4): in vivo Photosensitivity test

The following experiment was conducted with reference to the method of Gerberick et al. (Food Chem. Toxicol., 27, 813-819(1989)) with some modifications. First, ethanol was used to prepare a mixed solution containing the test substances shown in Tables 4 and 5 {*tert-*butylhydroxyanisole (BHA), di-*tert*-butylhydroxytoluene (BHT), propyl gallate} and 2% ketoprofen. As a control a 2% ketoprofen solution was prepared using ethanol.

Next, 20 µL of each prepared solution (N=8) was applied onto the auricle of a Balb/c mouse (female, 9-11 weeks old), prior to irradiation with 40 J/cm² UVA. When 24 hours had passed after UVA irradiation, the ear thickness of the mouse was measured and the increase with respect to the initial ear thickness was calculated. The degree of inhibition of ketoprofen-associated photosensitivity by each of the substances shown in Tables 4 and 5 was evaluated as the auricular edema inhibition (%) with respect to the ketoprofen group (control), representing the degree of inhibition of ketoprofen-induced ear thickness increase by each test substance. The obtained results are shown in Tables 4 and 5. The abbreviation "PG" in the tables stands for "propyl gallate".

**(Table 4)**

| | Comp. Example 1 | Comp. Example 2 | Example 4 |
|---|---|---|---|
| Test substance | 1.0% BHT | 0.2% PG | 1.0% BHT + 0.2% PG |
| Auricular edema inhibition (%) | 16% | 21% | 89% |

**(Table 5)**

| | Comp. Example 3 | Comp. Example 4 | Example 5 |
|---|---|---|---|
| Test substance | 0.5% BHA | 0.2% PG | 0.5% BHA + 0.2% PG |
| Auricular edema inhibition (%) | 24% | 19% | 84% |

As is clear from the results shown in Tables 4 and 5, combining an alkyl ester of gallic acid and BHA or BHT in a transdermal formulation for external application comprising ketoprofen as an active ingredient was confirmed to notably reduce photosensitivity through their synergistic action.

### (Example 6)

After adding a mixed solution of 75 µM ketoprofen and different concentrations of propyl gallate to a culturing medium (EpiLife™ Senim Free Medium E0151) (Cascade Biologics, Inc.) containing HPV16 immobilized human adult keratinocytes (KERTr) KEK001 (ATCC Co., Rockville, MD), the mixture was incubated for 2 hours at 37°C in 5% carbon dioxide/ 95% air, and then irradiated with 6-8 J/cm² UVA. When 24 hours had passed after UVA irradiation, the IL-1α concentration of the culture supernatant was evaluated with an ELISA kit (R&D Systems, Inc.). As a control, there was used the above-mentioned culturing medium to which a solution of 75 µM ketoprofen alone was added, which was incubated for 2 hours at 37°C in 5% carbon dioxide/ 95% air, and which was not irradiated with UVA.

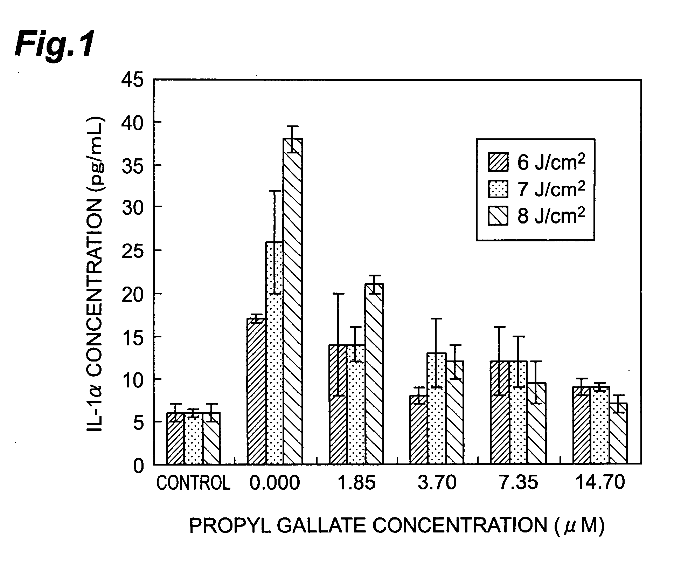

Fig. 1 is a graph showing the relationship between propyl gallate concentration in the mixed solution and IL-1α concentration in the culture supernatant, and it clearly indicates that an increased propyl gallate concentration reduces the IL-1α concentration. The experimental results shown in Fig. 1 demonstrated that IL-1α production is inhibited by propyl gallate even under UVA irradiation. They also demonstrated that the IL-1α production-inhibiting action of propyl gallate is adequately exerted even under strong UVA irradiation.

### (Example 7)

An experiment was conducted in the same manner as Example 6 using di-*tert*-butylhydroxytoluene (BHT) instead of propyl gallate.

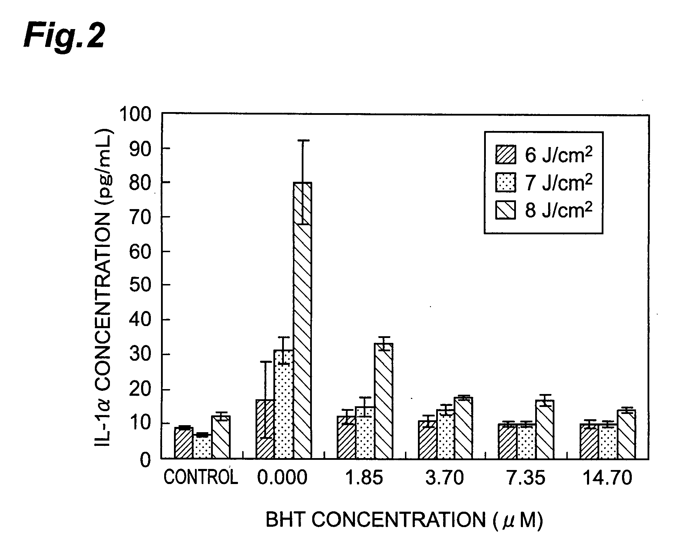

Fig. 2 is a graph showing the relationship between BHT concentration in the mixed solution and IL-1α concentration in the culture supernatant, and it indicates that an increased BHT concentration reduces the IL-1α concentration. The experimental results shown in Fig. 2 demonstrated that IL-1α production is inhibited by BHT even under UVA irradiation. They also demonstrated that the IL-1α production-inhibiting action of BHT is adequately exerted even under strong UVA irradiation.

### Industrial Applicability

As explained above, according to the present invention it is possible to reliably prevent photosensitivity in a transdermal formulation for external application comprising a non-steroidal anti-inflammatory analgesic as an active ingredient which can potentially induce photosensitivity, while still making it exert its anti-inflammatory analgesic action. It is also possible to inhibit dermatitis through its IL-1α production-inhibiting action.

## Claims

1. A transdermal formulation for external application comprising a non-steroidal anti-inflammatory analgesic, an alkyl ester of gallic acid, and a phenolic radical scavenger having a branched-chain lower alkyl group.

2. The transdermal formulation for external application according to claim 1, wherein said phenolic radical scavenger is at least one selected from the group consisting of *tert*-butylhydroxyanisole, di-*tert*-butylhydroxytoluene and thymol.

3. The transdermal formulation for external application according to claim 1 or 2, wherein said alkyl ester of gallic acid is an ester of gallic acid and a lower alcohol.

4. The transdermal formulation for external application according to any one of claims 1 to 3, wherein said non-steroidal anti-inflammatory analgesic is at least one selected from the group consisting of ketoprofen, tiaprofen, suprofen, tolmetin, carprofen, benoxaprofen, piroxicam, benzydamine, naproxen, diclofenac, ibuprofen, diflunisal and azapropazone.

5. An interleukin-1α production inhibitor consisting of a phenolic radical scavenger having a branched-chain lower alkyl group, and/or an alkyl ester of gallic acid.

6. The interleukin-1α production inhibitor according to claim 5, wherein said phenolic radical scavenger is at least one selected from the group consisting of *tert*-butylhydroxyanisole, di*-tert-*butylhydroxytoluene and thymol.

7. The interleukin-1α production inhibitor according to claim 5 or 6, wherein said alkyl ester of gallic acid is an ester of gallic acid and a lower alcohol.

8. The interleukin-1α production inhibitor according to any one of claims 5 to 7, which is used as a component in a transdermal formulation for external application comprising a non-steroidal anti-inflammatory analgesic.

9. The interleukin-1α production inhibitor according to claim 8, wherein said non-steroidal anti-inflammatory analgesic is at least one selected from the group consisting of ketoprofen, tiaprofen, suprofen, tolmetin, carprofen, benoxaprofen, piroxicam, benzydamine, naproxen, diclofenac, ibuprofen, diflunisal and azapropazone.

10. A transdermal formulation for external application comprising the interleukin-1α production inhibitor according to any one of claims 5 to 9 and a non-steroidal anti-inflammatory analgesic.

11. The transdermal formulation for external application according to claim 10, wherein said non-steroidal anti-inflammatory analgesic is at least one selected from the group consisting of ketoprofen, tiaprofen, suprofen, tolmetin, carprofen, benoxaprofen, piroxicam, benzydamine, naproxen, diclofenac, ibuprofen, diflunisal and azapropazone.

12. Use of the following substance (a) and/or substance (b) for inhibition of interleukin-1α production.
(a) A phenolic radical scavenger having a branched-chain lower alkyl group
(b) An alkyl ester of gallic acid

13. Use of the following substance (a) and/or substance (b) as a component in a transdermal formulation for external application comprising a non-steroidal anti-inflammatory analgesic.
(a) A phenolic radical scavenger having a branched-chain lower alkyl group
(b) An alkyl ester of gallic acid
